# EUROPEAN PATENT APPLICATION

(11) **EP 1 384 670 A1**
(43) Date of publication of application: **28.01.2004**
(21) Application number: 02722792.5
(22) Date of filing: 25.04.2002
(51) Int. Cl.: B65B 55/10

(54) **METHOD AND SYSTEM FOR STERILIZING FOOD PACKAGING CONTAINER OR FOOD FILLING SYSTEM**

(30) Priority: 01.05.2001 JP 2001133890; 01.05.2001 JP 2001133891; 01.05.2001 JP 2001133892
(71) Applicant: TOYO SEIKAN KAISYA, LTD., Tokyo 100-8522 (JP); Kao Corporation, Tokyo 103-8210 (JP)
(72) Inventor: IWASHITA, Takeshi, TOYO SEIKAN KAISHA, LTD., Yokohama-shi, Kanagawa 230-0001 (JP); SUNOHARA, Chikako, TOYO SEIKAN KAISHA, LTD., Yokohama-shi, Kanagawa 230-0001 (JP); SAKAI, Shigeru, TOYO SEIKAN KAISHA, LTD., Yokohama-shi, Kanagawa 230-0001 (JP); YOSHIKAWA, Kiyoaki, Kao Corporation, Wakayama-shi, Wakayama 640-8580 (JP); OKANO, Tetsuya, Kao Corporation, Wakayama-shi, Wakayama 640-8580 (JP); MATSUO, Noboru, Kao Corporation, Wakayama-shi, Wakayama 640-8580 (JP); TAMURA, Shigeru, .ao Corporation, Wakayama-shi, Wakayama 640-8580 (JP)
(74) Representative: HOFFMANN EITLE
(86) International application number: PCT/JP2002/004157
(87) International publication number: WO 2002/090188

(57) **Abstract**

The present invention provides a method of sterilizing a food packaging container or a food charging system, which comprises a chemical sterilizing treatment which involves contacting an aqueous sterilizing solution containing a hypochlorous acid-containing, chlorine-based sterilizing agent and a pH adjusting agent with the surface of a food packaging container or the surface of a line pipe, an instrument and a chamber in a food packaging system and a heat sterilizing treatment which involves increasing the temperature of the surface to be sterilized, the two sterilizing treatments being carried out simultaneously or in separate steps. The invention also provides a sterilizing device which has a predetermined sterilizing effect on a wide variety of microbial species, can be easily handled, does not corrode a treated surface over a prolonged period of time, is free of a pungent smell and does not require any special treatment for disposal of waste liquor.

## Description

### Technical Field

The present invention relates to a method of sterilizing the surfaces of food packaging containers such as PET bottles and plastic cups charged with various foods and drinks such as drinking water, juice, oolong tea and milk coffee and the surface of a line pipe, an instrument or a chamber in a food charging system and to a device therefor, and in particular to a method which has a predetermined sterilizing effect on a wide variety of microbial species, can be easily handled and does not corrode the surface of a line pipe, an instrument or a chamber in a food charging system over a prolonged period and to a device therefor. Further, the present invention relates to a sterilizing method which does not generate a pungent smell and does not require any special treatment for disposal of waste liquor.

### Background Art

Conventionally, when PET bottles are to be sterilized with a peracetic acid-based sterilizing agent compounded with hydrogen peroxide, the PET bottles are sterilized by charging the bottles with the sterilizing agent. After this sterilization, the charged sterilizing agent is discharged from the PET bottles, and the PET bottles are washed with aseptic water to remove the sterilizing agent from the PET bottles (washing step).

To prevent hydrogen peroxide or peracetic acid from remaining after the washing step, there is demand for reducing the concentration of hydrogen peroxide and peracetic acid used to lower levels. However, when the concentration of the hydrogen peroxide and peracetic acid used are reduced to lower levels, the sterilizing agent cannot demonstrate a potent sterilizing effect, and for sufficient sterilization, there inevitably arises the economical problem of a longer sterilization time.

As the method of sterilizing PET bottles to solve the problem in the prior art, there is an invention described in Japanese Patent No. 3080347. The sterilization method of this prior art invention comprises heating, at 60°C or more, a peracetic acid-based sterilizing agent containing peracetic acid at a concentration of 1000 to 1500 ppm and further containing hydrogen peroxide and then jetting the sterilizing agent via a nozzle onto at least the internal surface of a PET bottle.

By heating the sterilizing agent at 60°C or more, the invention described in Japanese Patent No. 3080347 supra could succeed in sterilizing PET bottles in a shorter time without increasing the concentration of the sterilizing agent. The sterilizing agent used in this invention, however, is a highly acidic, peracetic acid-based sterilizing agent compounded with hydrogen peroxide, so that after sterilization of a food charging system, the resulting waste liquor should be treated for disposal by reducing treatment, pH treatment or the like, resulting in the problem of higher costs. Further, the sterilizing agent is based on peracetic acid and thus has a pungent smell to make its storage and handling difficult, and it cannot be easily handled.

As a conventional method of cleansing and sterilizing a food packaging container such as PET bottle, there is known a method of contacting hot water with the surface of a food packaging container or a method of contacting a sterilizing agent such as hydrogen peroxide therewith. Further, JP-A 7-291236 discloses that a hot-water sterilizing step with hot water at 63°C or more is combined with a chemical sterilizing step with a sterilizing agent selected from the group consisting of hydrogen peroxide, peracetic acid, a mixture of hydrogen peroxide and peracetic acid, and sodium hypochlorite, and also that this sterilizing method is effective in sterilizing food containers.

The invention in JP-A 7-291236 supra can kill microorganisms without using a large amount of the sterilizing chemical. The sterilizing agent used in this invention, however, is a peracetic acid-based sterilizing agent which is so acidic that after sterilization of a food charging system, the resulting waste liquor should be treated for disposal by reducing treatment, pH treatment or the like, resulting in the problem of higher costs. Further, the sterilizing agent has a pungent smell to make its storage and handling difficult, and cannot be easily handled.

In recent studies, it has been revealed that this sterilizing agent, when used in a conventional method, is not so effective against some microbial species. Accordingly, when there is demand for an effective sterilizing effect on such microbial species, the chemical should be used in a larger amount, and therefore there arises problems such as very difficult treatment of the resulting waste liquor, etc.

The present invention is made to solve the above-described problems in the prior art, and the object of the present invention is to provide a method capable of sterilizing the surface of a food packaging container or a line pipe, an instrument and a chamber in a food packaging system in a shorter time without increasing the concentration of a sterilizing agent and without causing a pungent smell and problems such as an adverse effect on a line pipe, an instrument and a chamber wall in a food packaging system, as well as a device therefor. Another object of the present invention is to provide a novel sterilizing method which does not require any special treatment for disposal of waste liquor, and can limit successive introduction of a sterilizing agent under heating to the minimum level.

### Disclosure of Invention

The present invention relates to a method of sterilizing a food packaging container or a food charging system, which comprises a chemical sterilizing treatment which involves contacting an aqueous sterilizing solution containing a hypochlorous acid-containing, chlorine-based sterilizing agent and a pH adjusting agent with the surface of a food packaging container or the surface of a line pipe, an instrument and a chamber in a food packaging system and a heat sterilizing treatment which involves increasing the temperature of the surface to be sterilized, the two sterilizing treatments being carried out simultaneously or in separate steps.

The present invention comprises a chemical sterilizing treatment element and a heat sterilizing treatment element which can be carried out in separate steps or in the same step.

Preferably, the aqueous sterilizing solution has a pH value of 4 to 8, and is contacted in a heated state at 40°C to 80°C.

Particularly preferably, the chemical sterilizing treatment and the heat sterilizing treatment are carried out simultaneously.

According to another aspect of the invention, the chemical sterilizing treatment which involves contacting an aqueous sterilizing solution containing a hypochlorous acid-containing, chlorine-based sterilizing agent and a pH adjusting agent may be combined with the heat sterilizing treatment which involves increasing the temperature of the surface to be sterilized.

Preferably, the pH of the aqueous sterilizing solution is 4 to 8, and the heat sterilizing treatment is carried out at a temperature of 63°C or more.

The method of the present invention may further comprise contacting a surfactant.

The hypochlorous acid-containing, chlorine-based sterilizing agent is preferably at least one selected from hypochlorous acid and hypochlorite. The effective chlorine concentration of the hypochlorous acid-containing, chlorine-based sterilizing agent is preferably 200 to 2000 ppm.

The pH adjusting agent is preferably at least one member selected from the group consisting of alkali metal hydroxides, alkaline earth metal hydroxides, inorganic acids or salts thereof and organic acids or salts thereof.

The surfactant is preferably a surfactant derived from a polyvalent alcohol. The surfactant is preferably at least one member selected from the group consisting of polyglycerol fatty ester, glycerin fatty ester, sucrose fatty ester, sorbitan fatty ester, calcium stearoyllactate, propylene glycol fatty ester, lecithin such as soybean lecithin, yolk lecithin and plant lecithin, soybean saponin and beet saponin.

The heat sterilizing step can be carried out by using at least one of hot water, steam, hot air and a hot pack.

The heat sterilizing step can be carried out as a pre-step of the chemical sterilizing step, and the heat sterilizing step can be carried out by using at least one of hot water, steam and hot air. Further, the heat sterilizing step can be carried out as a post-step of the chemical sterilizing step, and the heat sterilizing step can be carried out by using a hot pack.

Further, the present invention provides a device for sterilizing a food packaging container or a food charging system, comprising sterilizing agent-contacting means in which an aqueous sterilizing solution containing a hypochlorous acid-containing, chlorine-based sterilizing agent and a pH adjusting agent is contacted in a heated state with the surface of a food packaging container or the surface of a line pipe, an instrument or a chamber in a food charging system by jetting, dipping or spraying. In the device of the present invention, the hypochlorous acid-containing, chlorine-based sterilizing agent and the pH adjusting agent can be contacted in a state heated at 40°C to 80°C with the surface by jetting, dipping or spraying. Further, after the surface is contacted with the hypochlorous acid-containing, chlorine-based sterilizing agent, the surface contacted with the sterilizing agent can be washed with aseptic water by jetting, dipping or spraying. Further, a surfactant can also be contacted.

According to the present invention, the sterilizing agent used as an aqueous solution containing at least one selected from hypochlorous acid and hypochlorite, a surfactant and a pH adjusting agent can be contacted with the surface to be sterilized to sterilize the surface in a very short time without increasing the concentration of the sterilizing agent. Further, the pH of the chlorine-based sterilizing agent such as hypochlorous acid is regulated near neutrality, whereby a safe and excellent sterilizing effect can be achieved with suppressed generation of a chlorine gas, and further the pH of the sterilizing agent comprising a mixture of the chlorine-based sterilizing agent and a surfactant is near neutrality, and thus the sterilizing agent does not require any special treatment for disposal of waste liquor, and can be used in a food charging system with no or less generation of a pungent smell. In addition, the present invention can be applied not only to the surface of a food packaging container but also to the surface of a line pipe, an instrument and a chamber in a food charging system as the object of sterilization.

### Best Mode for Carrying Out the Invention

According to the present invention, a wide variety of microbial species can be killed without increasing the concentration of the sterilizing agent and without causing a pungent smell and problems such as an adverse effect on a line pipe, an instrument and a chamber in a food packaging system.

In particular, the maximum sterilizing effect can be achieved by regulating the pH value of the sterilizing agent near neutrality, and further the pH value of the sterilizing agent is near neutrality, and therefore the sterilizing agent does not require any particular device for disposal of waste liquor, and can be easily handled with less generation of a pungent smell. Further, the sterilizing agent can be applied not only to the surface of a food packaging container but also to the surface of a line pipe, an instrument and a chamber in a food charging system as the object of sterilization.

The heated chlorine-based sterilizing agent containing hypochlorous acid is preferably the one with less pungent smell and not requiring any special treatment for disposal of waste liquor. This sterilizing agent can be obtained from sodium hypochlorite, a chlorine gas, chloride of lime, and sodium dichloroisocyanurate and the like.

To regulate the pH value of the sterilizing agent near neutrality from the viewpoint of preventing generation of a chlorine gas and achieving a safe and excellent sterilizing effect, the pH value of the hypochlorous acid-containing, chlorine-based sterilizing agent of the present invention is regulated preferably in the range of pH 4 to 8 with a pH regulating agent. The pH value is preferably pH 5 to 8, more preferably pH 5 to 7, still more preferably pH 6 to 7. In particular, the pH value is regulated preferably just before the sterilizing agent is contacted with an object of sterilization. The pH regulating agent used for this purpose is preferably a food additive such as succinic acid, citric acid, gluconic acid or L-tartaric acid.

When the chemical sterilizing treatment and the heat sterilizing treatment are simultaneously carried out, the hypochlorous acid-containing, chlorine-based sterilizing agent of the present invention is heated preferably 40°C or more, more preferably over 50°C, most preferably in the range of 65°C to 80°C.

The effective chlorine concentration in the hypochlorous acid-containing, chlorine-based sterilizing agent of the present invention is preferably 200 to 2000 ppm, still more preferably 200 to 600 ppm.

The hypochlorous acid-containing, chlorine-based sterilizing agent of the present invention is contacted with the surface to be sterilized successively preferably for 10 seconds or more, more preferably for 60 to 120 seconds. The hypochlorous acid-containing, chlorine-based sterilizing agent of the present invention is made effective against a wide variety of microbial species by pH adjustment and heating, and it was revealed that for example, when Bacillus circulans is to be killed as an objective microorganism, the microorganism cannot be killed at ordinary temperatures even upon being contacted with the sterilizing agent for 120 seconds, but can be killed in a short time of about 60 seconds by previously heating the sterilizing agent at 65°C or more. To demonstrate a higher sterilizing effect, therefore, not only the pH adjustment and heating of the sterilizing agent but also the contact time should be sufficiently examined.

The object of sterilization in the present invention is the internal and external surfaces of food packaging containers such as PET bottles and plastic cups.

The object of sterilization in a PET bottle aseptic charging system is the surface of each of an aseptic water line in a bottle rinser, a cap in a cap sterilizing machine, an aseptic water line in a cap rinse shoot, an instrument arranged in a charging chamber and the chamber. Further, the present invention can also be applied not only to a PET bottle aseptic charging system, but also to an aseptic charging system such as a plastic-cup aseptic charging system and to a hot-pack charging system.

The sterilizing agent of the present invention is a hypochlorous acid-containing, chlorine-based sterilizing agent whose pH is regulated near neutrality, so that even if a small amount of the sterilizing agent remains on the internal surface of a food packaging container after sterilization, the sterilizing agent is not particularly harmful to the human body, and therefore the step of washing the food packaging container with aseptic water after the sterilizing step can be eliminated depending on the type of charged food, thus bringing about a significant effect on reduction in the production process and in production costs.

The sterilizing agent can be added with a small amount of a surfactant and used as a mixed sterilizing agent to further improve the sterilizing effect.

In another aspect of the sterilizing method of the invention, the sterilizing agent used as an aqueous solution comprising the sterilizing agent, the surfactant and the pH adjusting agent can be heated.

In another aspect of the invention, the pH adjusting agent is characterized by being at least one member selected from the group consisting of alkali metal hydroxides, alkaline earth metal hydroxides, inorganic acids or salts thereof and organic acids or salts thereof, and the pH value of the aqueous solution is characterized by being 4 to 8.

In another aspect of the invention, the surfactant is characterized by being a surfactant derived from a polyvalent alcohol.

In a still another aspect of the invention, the surfactant is characterized by being a surfactant which can be added to foods, and is characterized by being at least one member selected from the group consisting of polyglycerol fatty ester, glycerin fatty ester, sucrose fatty ester, sorbitan fatty ester, calcium stearoyllactate, propylene glycol fatty ester, lecithin such as soybean lecithin, yolk lecithin and plant lecithin, soybean saponin and beet saponin.

The sterilizing agent used in another aspect of the invention comprises a sterilizing agent used as an aqueous solution containing at least one selected from hypochlorous acid and hypochlorite, a surfactant and a pH adjusting agent. In the present invention, the "surfactant" means a polyvalent alcohol-derived surfactantfor improving wetting properties of the surface of a microorganism, and is composed of glycerin fatty acid ester, polyglycerol fatty ester, propylene glycol fatty ester, polypropylene glycol fatty ester, sucrose fatty ester, sorbitan fatty ester, alkyl polyglycoside, etc. The "surfactant which can be added to foods" means a surfactant allowable as a food additive by law. The surfactant which can be added to foods is divided roughly into a chemically synthesized surfactant and a naturally occurring surfactant, and the chemically synthesized surfactant which can be used includes polyglycerol fatty ester, glycerin fatty ester, sucrose fatty ester, sorbitan fatty ester, calcium stearoyllactate, propylene glycol fatty ester, etc., and the naturally occurring surfactant which can be used includes lecithin such as soybean lecithin, yolk lecithin and plant lecithin and soybean saponin, beet saponin, etc.

As the finally used aqueous solution containing at least one selected from hypochlorous acid and hypochlorite, use can be made of a chlorine-based sterilizing agent such as alkali metal hypochlorite, chloride of lime, or sodium dichloroisocyanurate, among which sodium hypochlorite is preferable.

The compounding ratio of the surfactant which can be added to foods to the chlorine-based sterilizing agent is determined suitably in consideration of the microbial species to be sterilized, the type of food to be charged, the type of packaging container, etc. For example, when the internal and external surfaces of a PET bottle for drinking water and the internal and external surfaces of a cap are sterilized in a PET bottle aseptic charging system, the ratio of the concentration of the surfactant : the effective chlorine concentration of the chorine-based sterilizing agent in the mixed sterilizing agent can be in the range of 1 : 20 to 1 : 1 to achieve an excellent sterilizing effect in a short time.

In another aspect of the invention, the mixed sterilizing agent is produced desirably by adding a pH adjusting agent to hypochlorous acid or a salt thereof to regulate the pH value of the chlorine-based sterilizing agent near neutrality and then mixing it with a surfactant if necessary. From the viewpoint of preventing generation of a chlorine gas and achieving a safe and excellent sterilizing effect, the pH value is preferably pH 4 to 8, more preferably pH 5 to 8, still more preferably pH 5 to 7, further more preferably pH 6 to 7. The pH adjusting agent used for this purpose is at least one member selected from the group consisting of alkali metal hydroxides, alkaline earth metal hydroxides, inorganic acids or salts thereof, and organic acids or salts thereof. The alkali metal includes alkali metal hydroxides, alkaline earth metal hydroxides, inorganic acids or salts thereof, and organic acids or salts thereof. Among these, organic acids or salts thereof are preferable from the viewpoint of improvement of the sterilizing effect. The alkali metal hydroxides and alkaline earth metal hydroxide include sodium hydroxide, potassium hydroxide, calcium hydroxide, etc. The inorganic acids or salts thereof includes hydrochloric acid, sodium sulfate, sodium nitrate, sodium chloride, sodium carbonate, potassium hydrogen carbonate, sodium hydrogen carbonate, potassium hydrogen carbonate, magnesium sulfate, magnesium nitrate, magnesium chloride, trisodium phosphate, tripotassium phosphate, disodium hydrogen phosphate, dipotassium hydrogen phosphate, sodium dihydrogen phosphate, potassium dihydrogen phosphate, sodium polyphosphate, etc. The organic acids or salts thereof includes saturated dibasic acids such as malonic acid, succinic acid, glutaric acid, adipic acid and sebacic acid or salts thereof, unsaturated dibasic acids such as fumaric acid and maleic acid or salts thereof, and citric acid, acetic acid, lactic acid, malic acid, tartaric acid and gluconic acid, etc. The pH adjusting agent is preferably a saturated dibasic acid or a salt thereof, more preferably a saturated dibasic acid containing 3 to 10 carbon atoms or a salt thereof, still more preferably succinic acid or a salt thereof from the viewpoint of the safety of the pharmaceutical preparation. From the viewpoint of improvement of the sterilizing effect, the pH adjusting agent is preferably an organic acid or a salt thereof, particularly preferably succinic acid, citric acid, gluconic acid or L-tartaric acid as a food additive.

The mixed sterilizing agent of the present invention is used preferably at an effective chlorine concentration of 1 to 5000 ppm, more preferably 5 to 2000 ppm, still more preferably 5 to 1000 ppm.

When the microbial species to be killed is for example Bacillus cereus, the sterilizing agent of the present invention can be used at ordinary temperatures to achieve a sufficient sterilizing effect by contacting it in a short time of about 10 seconds, but it was found that when the microbial species to be killed is for example Bacillus circulans or Chaetomium sp. , the microorganism can be sterilized in a short time of about 10 seconds by previously heating the sterilizing agent at 65°C or more. Accordingly, the sterilizing agent is previously heated preferably at 65°C or more, more preferably at 65 to 100°C in order to exhibit a sufficient sterilizing effect on a wide variety of microbial species.

When the sterilizing agent of the present invention is used as an aqueous solution containing a combination of at least one selected from hypochlorous acid and hypochlorite, a surfactant which can be added to foods, and a pH adjusting agent, there is no particular harm to the human body even if a very small amount of the sterilizing agent remains on the internal surface of a food packaging container after sterilization, and therefore the step of washing the food packaging container with aseptic water after the sterilizing step can be eliminated depending on the type of charged food, thus bringing about a significant effect on reduction in the production process and in production costs.

In another aspect of the invention, the heat sterilizing treatment and the chemical sterilizing treatment are each carried out in separate steps. In the heat sterilizing step, the temperature of the surface to be sterilized is increased with a combination of one or two or more of hot water, steam, hot air and/or a hot pack.

For sterilizing every kind of microbial species sufficiently, heat sterilization is carried out desirably at a temperature of 63°C or more. That is, the surface to be sterilized is desirably heated to 63°C by contacting the surface to be sterilized with hot water, steam, hot air, a heated drink etc. A sufficient sterilizing effect on Bacillus circulans, Chaetomium sp. etc. cannot be achieved without heating the sterilizing agent in the chemical sterilizing step, but these microorganisms can be sufficiently killed by heating the surface to be sterilized at 63°C or more with hot water etc. in the heat sterilizing step. Accordingly, when Bacillus circulans or Chaetomium sp. is to be killed, the sterilizing agent may be used at ordinary temperatures (20 to 50°C) in the chemical sterilizing step insofar as the heat sterilization is carried out at a temperature of 63°C or more.

The heat sterilizing step using a combination of one or two or more of hot water, steam and hot air may be carried out as a pre- or post-step of the chemical sterilizing step. The heat sterilizing step may be carried out as a post-step by using a hot pack (hot charge). The hot pack is convenient because it is not necessary to arrange a special heating unit except for a previously arranged hot pack unit.

The mixed sterilizing agent for sterilization in a separate step in the present invention is used preferably at an effective chlorine concentration of 1 to 5000 ppm, more preferably 5 to 2000 ppm, still more preferably 5 to 1000 ppm.

As described above, microbial species such as Chaetomium sp. are killed by heat sterilization at 63°C or more in the heat sterilizing step, and thus the sterilizing agent of the present invention can be used at ordinary temperatures (20°C to 50°C).

### Technical Advantages of the Invention

According to the present invention, the hypochlorous acid-containing chlorine-based sterilizing agent in a state regulated in the range of pH 4 to 8 and heated at 40°C or more is contacted with the surface to be sterilized thereby effecting sterilization in a very short time without increasing the concentration of the sterilizing agent, as described above. By regulating the sterilizing agent in the range of pH 4 to 8, the sterilizing agent can achieve the maximum sterilizing effect, does not require any special device for treatment of waste liquor, and can be handled easily with less generation of a pungent smell. The sterilizing agent can be applied not only to the surface of a food packaging container, but also to the surface of a line pipe, an instrument or a chamber in a food charging system.

The sterilizing agent of the present invention is not harmful to the human body, and therefore the step of washing a food packaging container with aseptic water after the sterilizing step can be eliminated depending on the type of charged food, which can contribute to reduction in the production process and in production costs.

According to another aspect of the present invention, the sterilizing agent used as an aqueous solution containing at least one selected from hypochlorous acid and hypochlorite, a surfactant and a pH adjusting agent is contacted with the surface to be sterilized thereby effecting sterilization in a very short time without increasing the concentration of the sterilizing agent. By regulating the sterilizing agent consisting of a mixture of a chlorine-based sterilizing agent and a surfactant near neutrality, the sterilizing agent does not require any special treatment for disposal of waste liquor and can be used in a food charging system with no or less generation of a pungent smell. The sterilizing agent can be applied not only to the surface of a food packaging container, but also to the surface of a line pipe, an instrument or a chamber in a food charging system.

Further, the sterilizing agent of the present invention can, depending on the type of charged food, eliminate the step of washing a food packaging container with aseptic water after the sterilizing step, which can contribute to reduction in the production process and in production costs.

According to another aspect of the present invention, the sterilizing agent contains at least one selected from hypochlorous acid and hypochlorite, a surfactant and a pH adjusting agent, and therefore the pH of the chlorine-based sterilizing agent such as hypochlorous acid is regulated near neutrality, and the sterilizing agent as a mixture of this sterilizing agent and a surfactant is near neutrality, is thus free of a pungent smell and does not require any special treatment for disposal of waste liquor. Further, the heat sterilizing step is carried out as a pre- or post-step of the chemical sterilizing step so that even a microbial species which unless the sterilizing agent is heated, cannot be killed with the sterilizing agent can be killed in the heat sterilizing step, and thus heating of the sterilizing agent is not necessary. Accordingly, decomposition reaction due to heating of the sterilizing agent does not occur, and a wide variety of microbial species can be killed in a short time without requiring successive introduction of the sterilizing agent, and thus the amount of the chemical used can be limited to the minimum level.

### Brief Description of the Drawings

Fig. 1 is a diagram showing one embodiment of the device for carrying out the method of the present invention;
Fig. 2 is a sectional view showing one example of the method of sterilizing a plastic bottle in the present invention; and
   Numerals in these drawings are as follows:
   5: bottle sterilizing machine (unit for contacting a sterilizing agent)
   6: bottle rinser
   7: filler
   8: temporarily capping machine
   9: capper
Fig. 3 is a schematic diagram showing another example of the sterilization method of the present invention.
   Numerals in the drawing are as follows:
   101: bottle delivery unit
   102: bottle hot water sterilization zone
   103: bottle chemical sterilization zone
   104: rinse zone
   105: filling and sealing zone

### Description of Embodiments of the Invention and Examples

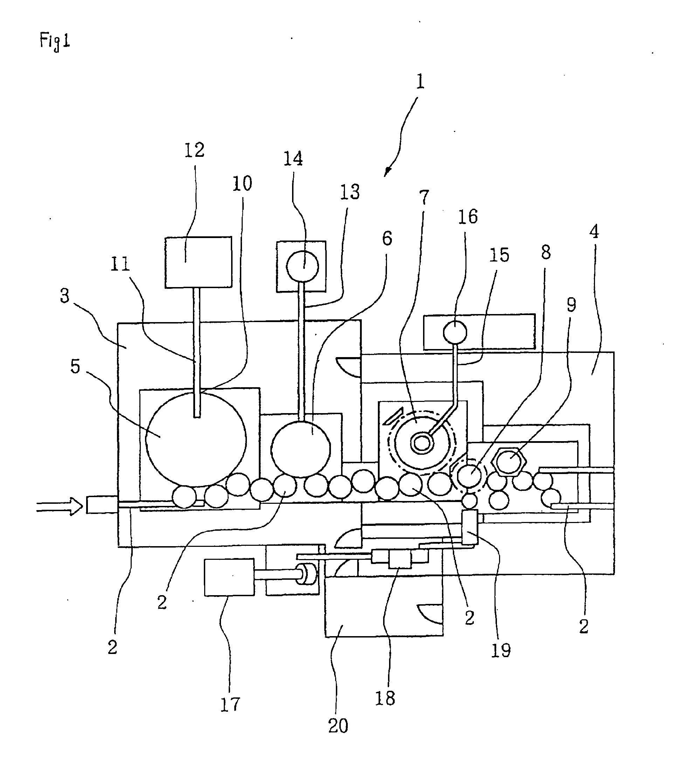

Hereinafter, one embodiment of the unit for carrying out the present sterilization method applied to a PET bottle aseptic charging system is described by referring to Fig. 1.

In Fig. 1, a PET bottle aseptic charging system 1 includes a PET bottle delivery unit 2 composed of a belt conveyer etc. and a sterilizing chamber 3 and a charging chamber 4 from the upstream side to the downstream side in the direction of bottle delivery. The sterilizing chamber 3 and the charging chamber 4 are each maintained at the germfree level of class 10000. The sterilizing chamber 3 is provided with a bottle sterilizing machine 5 and a bottle rinser 6 from the upstream side, and the charging chamber 4 is provided with a filler 7, a temporarily capping machine 8 and a capper 9 from the upstream side. Note that, reference numeral 20 is an operation room.

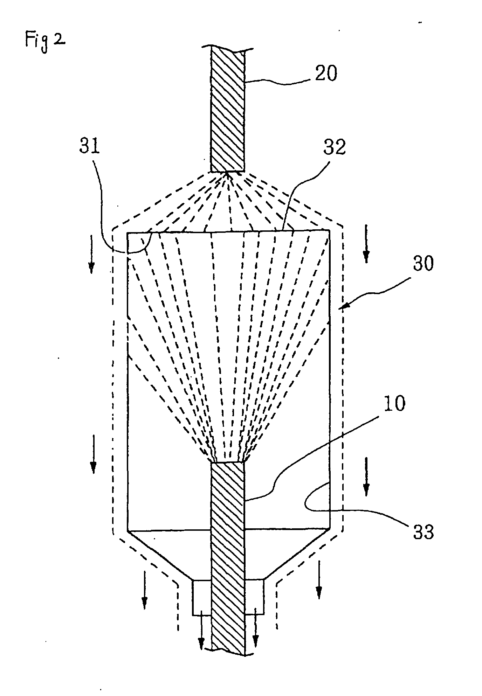

The bottle sterilizing machine 5 constituting means of contacting a sterilizing agent in the sterilizing device of the present invention sterilizes the internal surface and both of the internal and the external surfaces of a PET bottle, and allows a hypochlorous acid-containing, chorine-based sterilizing agent to be brought into contact with the surface of a PET bottle to be sterilized. The sterilizing agent is supplied from a sterilizing agent-feeding tank 12 via a pipe 11 to the bottle sterilizing machine 5, and in the bottle sterilizing machine 5 as shown in Fig. 2, surfaces such as a bottom internal surface 31, a bottom external surface 32 and a wall internal surface 33 of a PET bottle 30 in an inverted state are sterilized by jetting the sterilizing agent via first and second nozzles 10 and 20 attached to the pipe 11. Alternatively, the bottle sterilizing machine 5 may be constituted such that the PET bottle is sterilized by dipping the bottle in a sterilizing agent bath charged with the sterilizing agent or by spraying the surface of the PET bottle to be sterilized with the sterilizing agent. The sterilization time is preferably 10 to 120 seconds.

The bottle rinser 6 is supplied via a pipe 13 with aseptic water from an aseptic water tank 14 arranged in a germfree atmosphere. The bottle rinser 6 is provided with an aseptic water-jetting nozzle (not shown), and aseptic water is jetted to the PET bottle after sterilization, to wash out the sterilizing agent remaining on the surface of the PET bottle. Thereafter, the PET bottle in an inverted state is turned upside down and simultaneously fed to a filler 7. The bottle rinser 6, pipe 13, aseptic water tank 14 and aseptic water-jetting nozzle constitute means of contacting the PET bottle with aseptic water in the device of the present invention.

The filler 7 is a device for charging the PET bottle with a drink such as drinking water or juice, and is supplied with a drink via a pipe 15 from a drink tank 16. PET bottle caps discharged from a cap-feeding device 17, sterilized with a mixed sterilizing agent in a cap sterilizing machine 18 and washed with a cap rinse shoot 19 are supplied to a temporarily capping machine 8. In the temporarily capping machine 8, a supplied cap is mounted on the head of the drink-containing PET bottle just after being discharged from the filler, and the cap is temporarily attached thereto by light rotation and then sent to a capper 9. In the capper 9, the cap is fixed to the PET bottle by tightly turning the cap.

The PET bottle is supplied by the PET bottle delivery unit 2 from a PET bottle source (not shown) to the bottle sterilizing machine 5 in the sterilizing chamber 3, and the surface of the PET bottle to be sterilized is contacted for a predetermined time with the sterilizing agent by jetting, dipping or spraying. After sterilization, the PET bottle is sent to the downstream bottle rinser 6 where the sterilizing agent is washed out with aseptic water. Then, the PET bottle is sent to the filler 7 in the charging chamber 4 and charged with a drink. The PET bottle charged with a drink is temporarily fit with a cap in the temporarily capping machine 8, tightly fit with the cap finally in the capper 9, and delivered to the outside of the system.

The foregoing is an embodiment of the present invention applied to a PET bottle aseptic charging system, and when the present invention is applied to a plastic cap charging system, the PET bottle may be changed to a plastic cup, and the cap to a lid material, and the capper to a sealer (the temporarily capping machine is not necessary).

Hereinafter, one embodiment of the unit for carrying out the sterilizing method of the present invention applied to a PET bottle aseptic charging system is described by referring to Fig. 1.

In Fig. 1, a PET bottle aseptic charging system 1 includes a PET bottle delivery unit 2 composed of a belt conveyer etc. and a sterilizing chamber 3 and a charging chamber 4 from the upstream side to the downstream side in the direction of bottle delivery. The sterilizing chamber 3 and the charging chamber 4 are each maintained at the germfree level of class 10000. The sterilizing chamber 3 is provided with a bottle sterilizing machine (sterilizing agent-circulating device) 5 and a bottle rinser 6 from the upstream side, and the charging chamber 4 is provided with a filler 7, a temporarily capping machine 8 and a capper 9 from the upstream side. Note that, reference numeral 20 is an operation room.

The bottle sterilizing machine 5 constituting means of contacting the sterilizing agent of the present invention sterilizes the internal surface and both of the internal and the external surfaces of a PET bottle, and allows the mixed sterilizing agent of a surfactant which can be added to foods and a chlorine-based sterilizing agent to be brought into contact with the surface of a PET bottle to be sterilized. The mixed sterilizing agent is supplied from a sterilizing agent-feeding tank 12 via a pipe 11 to the bottle sterilizing machine 5, and in the bottle sterilizing machine 5 as shown in Fig. 2, surfaces such as a bottom internal surface 31, a bottom external surface 32 and a wall internal surface 33 of a PET bottle 30 in an inverted state are sterilized by jetting the sterilizing agent via first and second nozzles 10 and 20 attached to the pipe 11. Alternatively, the bottle sterilizing machine 5 may be constituted such that the PET bottle is sterilized by dipping the bottle in a mixed sterilizing agent bath charged with the mixed sterilizing agent or by spraying the surface of the PET bottle 30 with the mixed sterilizing agent. The sterilization time is preferably 10 to 80 seconds. The PET bottle may be contacted in a non-inverted state with the mixed sterilizing agent.

The bottle rinser 6 is supplied via a pipe 13 with aseptic water from an aseptic water tank 14 arranged in a germfree atmosphere. The bottle rinser 6 is provided with an aseptic water-jetting nozzle (not shown), and aseptic water is jetted to the PET bottle after sterilization, to wash out the mixed sterilizing agent remaining on the surface of the PET bottle. Thereafter, the PET bottle in an inverted state is turned upside down and simultaneously fed to a filler 7.

The filler 7 is a device for charging the PET bottle with a drink such as drinking water or juice, and is supplied with a drink via a pipe 15 from a drink tank 16. PET bottle caps discharged from a cap-feeding device (sterilizing agent-circulating device) 17, sterilized with the mixed sterilizing agent in a cap sterilizing machine 18 and washed with a cap rinse shoot 19 are supplied to a temporarily capping machine 8. In the temporarily capping machine 8, a supplied cap is mounted on the head of the drink-containing PET bottle just after being discharged from the filler, and the cap is temporarily attached thereto by light rotation and then sent to a capper 9. In the capper 9, the cap is fixed to the PET bottle by tightly turning the cap.

The PET bottle is supplied by the PET bottle delivery unit 2 from a PET bottle source (not shown) to the bottle sterilizing machine 5 in the sterilizing chamber 3, and the surface of the PET bottle to be sterilized is contacted for a predetermined time with the mixed sterilizing agent by jetting, dipping or spraying. After sterilization, the PET bottle is sent to the downstream bottle rinser 6 where the mixed sterilizing agent is washed out with aseptic water. Then, the PET bottle is sent to the filler 7 in the charging chamber 4 and charged with a drink. The PET bottle charged with a drink is temporarily fit with a cap in the temporarily capping machine 8, tightly fit with the cap finally in the capper 9, and delivered to the outside of the system.

The foregoing is an embodiment of the present invention applied to a PET bottle aseptic charging system, and when the present invention is applied to a plastic cap charging system, the PET bottle may be changed to a plastic cup, and the cap to a lid material, and the capper to a sealer (the temporarily capping machine is not necessary).

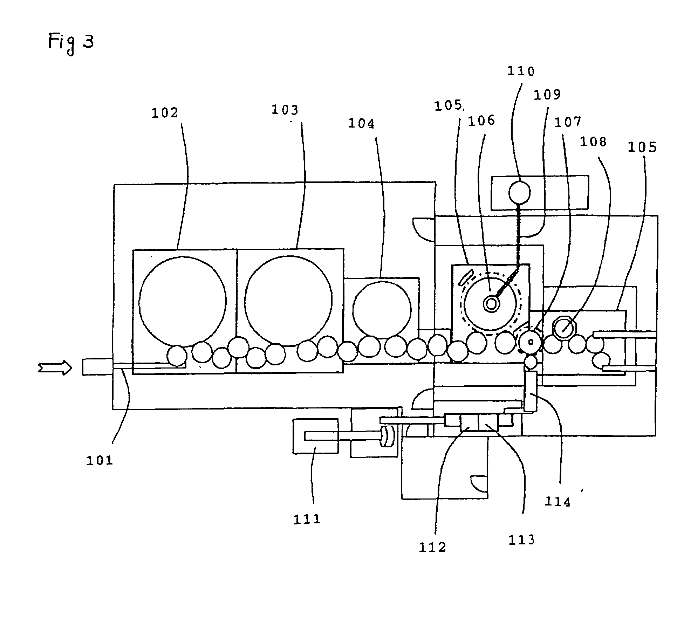

The sterilizing method of the present invention is described in more detail by referring to Fig. 3. Fig. 3 is a conceptual diagram showing one example of the method of sterilizing the internal surface of a bottle to be sterilized, and the heat sterilizing step is carried out with hot water. In Fig. 3, reference numeral 101 is a bottle delivery device composed of a belt conveyer etc., 102 is a bottle hot-water sterilizing zone, 103 is a bottle chemical sterilizing zone provided with a sterilizing agent-feeding nozzle, 104 is a rinsing region provided with an aseptic water-jetting nozzle, and 105 is a charging/sealing zone.

The bottle hot-water sterilizing zone 102 is provided with a nozzle capable of moving through the opening of a bottle, and from which hot water is jetted. The whole external surface of the bottle before sterilization is sterilized with hot water at 63°C or more and then delivered in an inverted state (with the opening facing downward) to the bottle hot-water sterilizing zone 102. In the bottle hot-water sterilizing zone 2, the nozzle is transferred into the opening of a bottle, and hot water at 63°C or more is jetted onto the internal surface of the bottle. Therefore, microbial species on the internal surface of the bottle are killed with hot water, and there remain only microbial species not killed with hot water.

The bottle sterilized in the bottle hot-water sterilizing zone 102 is delivered by the bottle delivery unit 101 to the bottle chemical sterilizing zone 103. In the bottle chemical sterilizing zone 103, the aqueous sterilizing solution is sprayed via a sterilizing agent-feeding nozzle into the bottle (spray system) , whereby the sterilizing agent is contacted with the whole internal surface of the bottle. The microorganisms not killed by the hot water are thereby killed, and all the microorganisms capable of growing in foods to be introduced into the bottle are killed. Because microorganisms to be hardly killed with the sterilizing agent are killed before sterilization with the sterilizing agent, the concentration of the sterilizing agent is reduced to a lower level. In the foregoing, sterilization with the sterilizing agent is carried out in a spray system, but the sterilizing agent may be stored in a tank to dip a bottle in the sterilizing agent or to fill it with the sterilizing agent. In the method described above, only the internal surface of the bottle is sterilized with the sterilizing agent, but as a matter of course, the external surface of the bottle may be sterilized with the sterilizing agent in order to achieve more reliable sterilization.

The bottle chemically sterilized with the sterilizing agent in the bottle chemical sterilizing zone 103 is delivered by the delivery unit 101 to a rinsing zone 104. In the rinsing zone 104, aseptic water at ordinary temperatures or aseptic hot water is jetted onto at least the internal surface of the bottle, whereby the sterilizing agent adhering to the bottle is removed from the surface of the bottle. The concentration of the sterilizing agent used is at a lower level as described above, and thus the concentration of the sterilizing agent adhering to the bottle sent to the rinsing zone 104 is low, and the sterilizing agent can be removed easily and certainly with a small amount of aseptic water or aseptic hot water.

After rinsing, the bottle in an inverted state is turned upside down by bottle-inverting means (not shown) , then sprayed with germfree air and simultaneously sent by the bottle delivery device 101 to a charging chamber, that is, a charging/sealing zone 5. The charging/sealing zone 5 is maintained at a level of class 100 or less where the bottle is charged with a food or drink such as milk coffee by a charging unit known per se. The charging/sealing zone 105 is provided with a filer 106, a temporarily capping machine 7 and a capper 108 from the upstream side.

The filler 106 is a device for charging a bottle with a drink such as drinking water, juice etc., and is supplied with a drink via a pipe 109 from a drink tank 110. Caps discharged from a cap-feeding device 111, sterilized with hot water in a cap hot-water sterilizing zone 112, chemically sterilized with the chemical in a cap chemical sterilizing zone 13 and washed with a cap rinse shoot 114 are supplied to a temporarily capping machine 107. In the temporarily capping machine 107, a supplied cap is mounted on the head of the drink-containing bottle just after being discharged from the filler 106, and the cap is temporarily attached thereto by light rotation and then sent to a capper 108. In the capper 108, the cap is fixed to the bottle by tightly turning the cap. Thereafter, the bottle is inspected in a product examination zone (not shown) to finish the whole process.

In the example in Fig. 3, the bottle hot-water sterilizing zone 102 is arranged before the bottle chemical sterilizing zone 3, so that hot-water sterilization can be carried out before chemical sterilization, but the bottle hot-water sterilizing zone 2 may be arranged after the bottle chemical sterilizing zone 103 so that hot-water sterilization can be carried out after chemical sterilization. In this case similar to the above case where chemical sterilization is first carried out, hot-water sterilization and chemical sterilization compensate for each other, and thus the concentration of the chemical can be reduced to a lower level. In addition, when hot-water sterilization is carried out after chemical sterilization, hot-water sterilization is carried out simultaneously with rinsing of the chemical used in the chemical sterilizing step and remaining on a food container, and the bottle treatment time including the time of sterilization and subsequent rinsing can be reduced.

The chemical can be used in a circulating system or spent up in one process.

In the above example, the heating step and sterilizing step are carried out in separate zones, but in the same zone, sterilization may be carried out after heating, or heating may be carried out after sterilization, or heating and sterilization can be simultaneously carried out (two nozzles are used for simultaneously spraying hot water and the chemical). In the last case, the system can be simplified.

### Examples

### (Example 1)

To demonstrate the effectiveness of the sterilizing method of the present invention, the sterilizing effect of the method on the internal surface of a PET bottle among the surfaces of food packaging materials, line pipes, instruments or chambers was examined as an example.

As a chlorine-based sterilizing agent, a pharmaceutical preparation (sterilizing agent) was prepared by diluting sodium hypochlorite with water and then adding a sufficient acid to the finally used solution to adjust its pH as described below.

A test microorganism was allowed to adhere to the internal surface of a PET bottle in an amount of 104 cfu/bottle (intended level), and the internal surface of the PET bottle was sterilized under test conditions described later. After sterilization, the bottle was charged with a medium (microbial spores; standard liquid medium) , capped with an aseptic cap and stored for 1 week (microbial spores; standard liquid medium). After storage, the sterilizing effect on the adhering microorganism was determined by evaluating the medium with the naked eye. In the table, "+" indicates the presence of the adhering microorganism, while "-" indicates the absence of the adhering microorganism.

**Table 1**

| Sterilizing effect on Bacillus circulans Effective chlorine concentration :200ppm | | | | | | | |
|---|---|---|---|---|---|---|---|
| Test microorganisms | Number ofmicroorganisms adhering to the surface (cfu/cm2) | Temperature (°C) | pH | Sterilization time(second) | | | |
| | | | | 10 | 60 | 120 | 300 |
| *Bacillus circulans* circulans spores | 1. 2 × 10⁴ | 80 | 10 | + | + | + | + |
| | | | 8 | - | - | - | - |
| | | | 6 | - | - | - | - |
| | | | 4 | - | - | - | - |
| | | | 2 | + | + | + | + |
| | | 65 | 10 | + | + | + | + |
| | | | 8 | + | - | - | - |
| | | | 6 | + | - | - | - |
| | | | 4 | + | - | - | - |
| | | | 2 | + | + | + | + |
| | | 50 | 10 | + | + | + | + |
| | | | 8 | + | + | - | - |
| | | | 6 | + | + | - | - |
| | | | 4 | + | + | - | - |
| | | | 2 | + | + | + | + |
| | | 40 | 10 | + | + | + | + |
| | | | 8 | + | + | + | - |
| | | | 6 | + | + | + | - |
| | | | 4 | + | + | + | - |
| | | | 2 | + | + | + | + |
| | | 25 | 10 | + | + | + | + |
| | | | 8 | + | + | + | + |
| | | | 6 | + | + | + | + |
| | | | 4 | + | + | + | + |
| | | | 2 | + | + | + | + |

**Table 2**

| Sterilizing effect on Bacillus circulans spores Effective chlorine concentration: 400 ppm | | | | | | | |
|---|---|---|---|---|---|---|---|
| Test microorganisms | Number of microorganisms adhering to the surface (cfu/cm2) | Temperature (°C) | pH | Sterilization time (second) | | | |
| | | | | 10 | 60 | 120 | 300 |
| *Bacillus circulans* spores | 1. 2 × 10⁴ | 65 | 10 | + | + | + | + |
| | | | 8 | - | - | - | - |
| | | | 6 | - | - | - | - |
| | | | 4 | - | - | - | - |
| | | | 2 | + | + | + | + |
| | | 50 | 10 | + | + | + | + |
| | | | 8 | + | - | - | - |
| | | | 6 | + | - | - | - |
| | | | 4 | + | - | - | - |
| | | | 2 | + | + | + | + |
| | | 40 | 10 | + | + | + | + |
| | | | 8 | + | + | - | - |
| | | | 6 | + | + | - | - |
| | | | 4 | + | + | - | - |
| | | | 2 | + | + | + | + |
| | | 25 | 10 | + | + | + | + |
| | | | 8 | + | + | + | - |
| | | | 6 | + | + | + | - |
| | | | 4 | + | + | + | - |
| | | | 2 | + | + | + | + |

### Example 2-1

To confirm the effectiveness of the sterilizing method of the present invention, the sterilizing effect of the method on the internal surface of a PET bottle as an object of sterilization was examined.

As at least one selected from hypochlorous acid and hypochlorite, sodium hypochlorite was diluted with water and then adjusted to pH 6.3±0.3 with succinic acid as the pH adjusting agent. Among surfactants which can be added to foods, polyglycerol fatty ester was used as the surfactant and mixed therewith, and the mixed aqueous solution was used at the concentrations shown in Tables 3 and 4 . The effective chlorine concentrations in Tables 3 and 4 were determined by the iodine method JIS K-0101. In the comparative example, a sterilizing agent consisting of only an aqueous sodium hypochlorite solution (pH 10) whose pH was not regulated with a pH adjusting agent.

As the test microorganism, Bacillus cereus spores were allowed to adhere to the internal surface of a PET bottle in an amount of 106 cfu/bottle (intended level), and the internal surface of the PET bottle was sterilized under the test conditions shown in Table 3 below. After sterilization, the bottle was charged with a standard liquid medium, capped with an aseptic cap and stored for 1 week. After storage, the sterilizing effect on the adhering microorganism was determined by evaluating the medium with the naked eye. The results of determination are shown in Table 3. In the table, "+" indicates the presence of the adhering microorganism, while "-" indicates the absence of the adhering microorganism.

**Table 3**

| Sterilizing effect on Bacillus cereus spores, | | | | | | | | |
|---|---|---|---|---|---|---|---|---|
| Test microorganism | Number of microorganisms adhering to the surface (cfu/cm2) | polyglycerin aliphatic ester (ppm) | Effective chlorine (ppm) | Temperature (°C) | Sterilization time(second) | | | |
| | | | | | | | | |
| | | | | | 10 | 20 | 30 | 60 |
| *Bacillus cereus* spores | 5.0 × 10⁶ | 200 | 400 | 25 | - | - | - | - |
| | | | | 35 | - | - | - | - |
| | | | | 50 | - | - | - | - |
| | | | | 65 | - | - | - | - |
| | | | | 80 | - | - | - | - |
| | | 0 | 400 | 25 | + | + | + | + |
| | | | | 35 | + | + | + | + |
| | | | | 50 | + | + | + | + |
| | | | | 65 | + | + | + | + |
| | | | | 80 | + | + | + | + |

### Example 2-2

As test microorganisms, microbial spores i.e. Bacillus circulans spores and fungal spore i.e. Chaetomium sp. ascospores were selected, and the same mixed sterilizing agent as in Example 1 was used to sterilize PET bottles under the test conditions shown in Table 4 below. After sterilization, the bottle with the Bacillus circulans spores was charged with a standard liquid medium, the bottle with Chaetomium sp. ascospores was charged with a potato dextrose standard liquid medium, and each bottle was capped with an aseptic cap and stored for 1 week, and then the medium was determined with the naked eye. The results of determination are shown in Table 4.

**Table 4**

| Sterilizing effect on Bacillus circulans spores, and sterilizing effect on Chaetomium sp. ascospores | | | | | | | | |
|---|---|---|---|---|---|---|---|---|
| Test microorganism | Number of microorganisms adhering to the surface (cfu/cm2) | polyglycerin aliphatic ester (ppm) | Effective chlorine (ppm) | Temperature (°C) | Sterilizationtime(second) | | | |
| | | | | | 10 | 20 | 30 | 60 |
| *Bacillus circulans* spores | 7.0 × 10⁵ | 200 | 400 | 35 | + | + | + | + |
| | | | | 50 | + | + | + | - |
| | | | | 60 | + | + | - | - |
| | | | | 65 | - | - | - | - |
| | | | | 80 | - | - | - | - |
| *Chaetomium sp.* | 1.0 × 10⁶ | 200 | 400 | 35 | + | + | + | + |
| | | | | 50 | + | + | + | + |
| | | | | 60 | + | + | + | + |
| | | | | 65 | - | - | - | - |
| | | | | 80 | - | - | - | - |

### Example 3-1

(1) Fungi: genera Aspergillus, Penicillium, Byssochamys, Neosartorya and Chatomium.
(2) Yeasts: genera Saccharomyces and Candida.
(3) Microorganisms, genus Bacillus, group A: Bacillus subtilis var. niger and Bacillus subtilis.
(4) Microorganisms, genus Bacillus, group B: Bacillus cereus and Bacillus circulans.

Each of the microorganisms listed in (1) to (4) (microorganisms referred to as the group were mixed in equal amounts) was allowed to adhere to the internal surface of a test bottle in an amount of 105 to 106 cfu/bottle, and the bottle was fit with a cap. The test bottle was a PET (polyethylene terephthalate) bottle having an internal volume of 500 ml, and the test cap was a (polyethylene) cap for an aperture of 28. The internal surfaces of the test bottle and cap.were each sterilized with hot water in a system selected from those in item 1 below (after chemical sterilization in the case of 1-3) and then sterilized with a chemical in a system selected from those in item 2 below, and the adhering chemical was washed out with aseptic water, and the number of living microorganisms on the internal surface of the bottle was measured to determine the sterilizing effect (challenge test method). The results are shown in Table 5.

### 1. Hot-water sterilizing conditions

### 1-1. Hot-water spray system

(1) Sterilization system: a system of spraying a test bottle and a cap with hot water
(2) Hot-water temperature: 63°C (on the surfaces of the bottle and cap)
(3) Flow rate of hot water: 200 ml/sec.
(4) Sterilization time: 4 seconds with hot water
(5) Sterilization frequency: twice

### 1-2. Steam spray system

(1) Sterilization system: a system of spraying a test bottle and a cap with steam
(2) Steam temperature: 63°C (on the surfaces of the bottle and cap)
(3) Steam pressure: 1.2 kg/cm²
(4) Sterilization time: 10 seconds

### 1-3. Hot-pack charging system

(1) Sterilization system: a system for charging a test bottle with a heated content
(2) Charging temperature: 63°C (on the surface of the bottle)
(3) Charging rate: 100 ml/sec.
(4) Charging time: 5 seconds
(5) Time after charging: maintained in an inverted state for 30 seconds after capping.

### 2. Chemical sterilization conditions

### 2-1. Chemical spray sterilization system

(1) Sterilization system: a system of spraying a test bottle and a cap with a chemical
(2) Preparation of the chemical: Sodium hypochlorite is diluted with water and then adjusted to pH 6.3±0.3 with succinic acid as the pH adjusting agent. Polyglycerol fatty ester is used as the surfactant and mixed therewith, and the mixed aqueous solution is regulated at 200 ppm and used as the chemical.
(3) Effective chlorine concentration: the same as described in 2-1.
(4) Chemical temperature: 50°C
(5) Sterilization time: 1 minute

### 2-2. Chemical charging sterilization system

(1) Sterilization system: a system in which the chemical is introduced into a test bottle and a cap, maintained and discharged.
(2) Preparation of the chemical: the same as described in 2-1
(3) Effective chlorine concentration: 400 ppm
(4) Chemical temperature: 50°C
(4) Sterilization time: 1 minute

### Comparative Example 1

Hot-water sterilization only was carried out under the same conditions as in Example 3-1, and chemical sterilization was not conducted. The sterilizing effect is shown in Table 5.

### Comparative Example 2

Chemical sterilization only was carried out under the same conditions as in Example 3-1, and hot-water sterilization was not conducted. The sterilizing effect is shown in Table 5.

### Comparative Example 3

Hot-water sterilization only was carried out under the same conditions as in Example 1 except that the temperature of hot water was 40°C in place of 63°C, and chemical sterilization was not conducted. The sterilizing effect is shown in Table 5.

### Comparative Example 4

Hot-water sterilization and chemical sterilization were carried out under the same conditions as in Example 3-1 except that the temperature of hot water used in hot-water sterilization was 40°C in place of 63°C. The sterilizing effect is shown in Table 5.

### Comparative Example 5

A test bottle and a cap were sterilized in the same manner as in Example 3-1 except that the sterilization conditions were described below. The sterilizing effect is shown in Table 5. Sterilization system: A chemical is introduced into the test bottle and cap, maintained and discharged.
Preparation of the chemical: Oxonia 3% (peracetic acid concentration 1500 ppm)
Chemical temperature: 40°C
Sterilizing time: 3 minutes

### Comparative Example 6

A test bottle and a cap were sterilized in the same manner as in Example 3-1 except that the sterilization conditions were described below. The sterilizing effect is shown in Table 5. The chemical used was a sterilizing having a pH value of 10.
(1) Sterilization system: A chemical is introduced into the test bottle and cap, maintained and discharged.
(2) Preparation of the chemical: sodium hypochlorite
(3) Effective chlorine concentration: 100 ppm
(4) Chemical temperature: 50°C
(5) Sterilizing time: 3 minutes

**Table 5**

| Microbial species | Example 3-1 | Comparative example 1 | Comparative example 2 | Comparative example 3 | Comparative example 4 | Comparative example 5 | Comparative example 6 |
|---|---|---|---|---|---|---|---|
| Genus Aspergillus | ⓞ | ○ | ⓞ | × | ⓞ | ⓞ | ⓞ |
| Genus Penicillium | ⓞ | ⓞ | ⓞ | × | ⓞ | ⓞ | ⓞ |
| Genus Byssochamys | ⓞ | × | ⓞ | × | ⓞ | ⓞ | ⓞ |
| Genus Neosartorya | ⓞ | × | ⓞ | × | ⓞ | ⓞ | ⓞ |
| Genus Chaetomium | ⓞ | ⓞ | Δ | × | Δ | ⓞ | ⓞ |
| Genus Saccharomyces | ⓞ | ○ | ⓞ | × | ⓞ | ⓞ | ⓞ |
| Genus Candidia | ⓞ | ○ | ⓞ | × | ⓞ | ⓞ | ⓞ |
| Genus Bacillus, group A | ⓞ | × | ⓞ | × | ⓞ | ⓞ | ⓞ |
| Genus Bacillus, group B | ⓞ | × | ⓞ | × | ⓞ | × | × |

In Table 5, ⓞ indicates that no microorganism adhering to the test bottle was detected, ○ indicates that 100 to 101 cfu adhering microorganisms per test bottle were detected, Δ indicates that 101 to 103 cfu adhering microorganisms per test bottle were detected, and X indicates that 103 to 105 or more cfu adhering microorganisms were detected.

### Example 3-2

A test bottle and a cap were sterilized in the same manner as in Example 3-1 except that hot-water sterilization was carried out at 80°C in place of 63°C. The sterilizing effect is shown in Table 6.

### <Comparative Example 7>

Hot-water sterilization only was carried out under the same conditions as in Example 3-2, and chemical sterilization was not conducted. The sterilizing effect is shown in Table 6.

**Table 6**

| Microbial species | Example 3-2 | Comparative Example 7 |
|---|---|---|
| Genus Aspergillus | ⓞ | ⓞ |
| Genus Penicillium | ⓞ | ⓞ |
| Genus Byssochamys | ⓞ | ⓞ |
| Genus Neosartorya | ⓞ | × |
| Genus Chaetomium | ⓞ | ⓞ |
| Genus Saccharomyces | ⓞ | ⓞ |
| Genus Candidia | ⓞ | ⓞ |
| Genus Bacillus, group A | ⓞ | × |
| Genus Bacillus, group B | ⓞ | × |

In Table 6 similar to Table 5, ⓞ indicates that no microorganism adhering to the test bottle was detected, ○ indicates that 100 to 101 cfu adhering microorganisms per test bottle were detected, Δ indicates that 101 to 103 cfu adhering microorganisms per test bottle were detected, and × indicates that 103 to 105 or more cfu adhering microorganisms were detected.

### Example 3-3

A test bottle and a cap were sterilized in the same manner as in Example 3-1 except that hot-water sterilization was carried out at 93°C in place of 63°C. The sterilizing effect is shown in Table 7.

### <Comparative Example 8>

Hot-water sterilization only was carried out under the same conditions as in Example 3-3 except that chemical sterilization was not conducted. The sterilizing effect is shown in Table 7.

**Table 7**

| Microbial species | Example 3-3 | Comparative Example 8 |
|---|---|---|
| Aspergillus | ⓞ | ⓞ |
| Genus Penicillium | ⓞ | ⓞ |
| Genus Byssochamys | ⓞ | ⓞ |
| Genus Neosartorya | ⓞ | Δ |
| Genus Chaetomium | ⓞ | ⓞ |
| Genus Saccharomyces | ⓞ | ⓞ |
| Genus Candidia | ⓞ | ⓞ |
| Genus Bacillus, group A | ⓞ | × |
| Genus Bacillus, group B | ⓞ | × |

In Table 7 similar to Table 5, ⓞ indicates that no microorganism adhering to the test bottle was detected, ○ indicates that 100 to 101 cfu adhering microorganisms per test bottle were detected, Δ indicates that 101 to 103 cfu adhering microorganisms per test bottle were detected, and × indicates that 103 to 105 or more cfu adhering microorganisms were detected.

### Example 3-4

### <Example 3-1 (1) > A test bottle and a cap were sterilized in the same manner as in Example 3-1. The sterilizing effect is shown in Table 8.

### <Example 3-1 (2) > A test bottle and a cap were sterilized in the same manner as in Example 3-1 except that the effective chlorine concentration for chemical sterilization was 200 ppm in place of 400 ppm. The sterilizing effect is shown in Table 4.

**Table 8**

| Microbial species | Example 3-1① | Example 3-1② |
|---|---|---|
| Aspergillus | ⓞ | ⓞ |
| Genus Penicillium | ⓞ | ⓞ |
| Genus Byssochamys | ⓞ | ⓞ |
| Genus Neosartorya | ⓞ | ⓞ |
| Genus Chaetomium | ⓞ | ⓞ |
| Genus Saccharomyces | ⓞ | ⓞ |
| Genus Candidia | ⓞ | ⓞ |
| Genus Bacillus, group A | ⓞ | ⓞ |
| Genus Bacillus, group B | ⓞ | ⓞ |

In Table 8 similar to Table 5, ⓞ indicates that no microorganism adhering to the test bottle was detected.

**Table 9**

| Microbial species | pH10 | pH8 | pH6 | pH4 | pH2 |
|---|---|---|---|---|---|
| Aspergillus | ⓞ | ⓞ | ⓞ | ⓞ | ⓞ |
| Genus Penicillium | ⓞ | ⓞ | ⓞ | ⓞ | ⓞ |
| Genus Byssochamys | ⓞ | ⓞ | ⓞ | ⓞ | ⓞ |
| Genus Neosartorya | ⓞ | ⓞ | ⓞ | ⓞ | ⓞ |
| Genus Chaetomium | ⓞ | ⓞ | ⓞ | ⓞ | ⓞ |
| Genus Saccharomyces | ⓞ | ⓞ | ⓞ | ⓞ | ⓞ |
| Genus Candidia | ⓞ | ⓞ | ⓞ | ⓞ | ⓞ |
| Genus Bacillus, group A | ⓞ | ⓞ | ⓞ | ⓞ | ⓞ |
| Genus Bacillus, group B | × | ○ | ⓞ | ○ | × |

The pH value was varied under the same conditions as in Example 3-1 (effective chlorine concentration of the chemical: 400 ppm).

In Table 5, ⓞ indicates that no microorganism adhering to the test bottle was detected, ○ indicates that 100 to 101 cfu adhering microorganisms per test bottle were detected, Δ indicates that 101 to 103 cfu adhering microorganisms per test bottle were detected, and × indicates that 103 to 105 or more cfu adhering microorganisms were detected.

The same test was carried out at an effective chlorine concentration of 200 ppm, and the same sterilizing effect was confirmed.

## Claims

1. A method of sterilizing a food packaging container or a food charging system, which comprises a chemical sterilizing treatment which involves contacting an aqueous sterilizing solution containing a hypochlorous acid-containing, chlorine-based sterilizing agent and a pH adjusting agent with the surface of a food packaging container or the surface of a line pipe, an instrument and a chamber in a food packaging system and a heat sterilizing treatment which involves increasing the temperature of the surface to be sterilized, the two sterilizing treatments being carried out simultaneously or in separate steps.

2. The method according to claim 1, wherein the aqueous sterilizing solution has a pH value of 4 to 8, and is contacted in a heated state at 40°C to 80°C.

3. The method according to claim 2, wherein the chemical sterilizing treatment and the heat sterilizing treatment are carried out simultaneously.

4. The method according to claim 1, which comprises a chemical sterilizing treatment which involves contacting an aqueous sterilizing solution containing a hypochlorous acid-containing, chlorine-based sterilizing agent and a pH adjusting agent combined with a heat sterilizing treatment which involves increasing the temperature of the surface to be sterilized.

5. The method according to claim 4, wherein the pH of the aqueous sterilizing solution is 4 to 8, and the heat sterilizing treatment is carried out at a temperature of 63°C or more.

6. The method according to any one of claims 1 to 5, which further comprises contacting a surfactant.

7. The method according to any one claims 1 to 6, wherein the hypochlorous acid-containing, chlorine-based sterilizing agent is at least one selected from hypochlorous acid and hypochlorite.

8. The method according to any one of claims 1 to 7 , wherein the effective chlorine concentration of the hypochlorous acid-containing, chlorine-based sterilizing agent is 200 to 2000 ppm.

9. The method according to any one of claims 1 to 8, wherein the pH adjusting agent is at least one member selected from the group consisting of alkali metal hydroxides, alkaline earth metal hydroxides, inorganic acids or salts thereof and organic acids or salts thereof.

10. The method according to any one of claims 6 to 9, wherein the surfactant is a surfactant derived from a polyvalent alcohol.

11. The method according to any one of claims 6 to 10, wherein the surfactant is at least one member selected from the group consisting of polyglycerol fatty ester, glycerin fatty ester, sucrose fatty ester, sorbitan fatty ester, calcium stearoyllactate, propylene glycol fatty ester, lecithin such as soybean lecithin, yolk lecithin and plant lecithin, soybean saponin and beet saponin.

12. The method according to any one of claims 4 to 11, wherein the heat sterilizing step is carried out by using at least one of hot water, steam, hot air and a hot pack.

13. The method according to claim 12, wherein the heat sterilizing step is carried out as a pre-step of the chemical sterilizing step, and the heat sterilizing step is carried out by using at least one of hot water, steam and hot air.

14. The method according to claim 12, wherein the heat sterilizing step is carried out as a post-step of the chemical sterilizing step, and the heat sterilizing step is carried out by using a hot pack.

15. A device for sterilizing a food packaging container or a food charging system, comprising sterilizing agent-contacting means in which an aqueous sterilizing solution containing a hypochlorous acid-containing, chlorine-based sterilizing agent and a pH adjusting agent is contacted in a heated state with the surface of a food packaging container or the surface of a line pipe, an instrument or a chamber in a food charging system by jetting, dipping or spraying.

16. The device according to claim 15, wherein the hypochlorous acid-containing, chlorine-based sterilizing agent and the pH adjusting agent are contacted in a state heated at 40°C to 80°C with the surface by jetting, dipping or spraying.

17. The device according to claim 16, wherein the surface is contacted with the hypochlorous acid-containing, chlorine-based sterilizing agent, and then the surface contacted with the sterilizing agent is washed with aseptic water by jetting, dipping or spraying.

18. The device according to any one of claims 15 to 17, wherein a surfactant is further contacted.
